# EUROPEAN PATENT APPLICATION

(11) **EP 2 221 050 A1**
(43) Date of publication of application: **25.08.2010**
(21) Application number: 08850531.8
(22) Date of filing: 14.11.2008
(51) Int. Cl.: A61K 9/50, C08B 37/04, C08G 63/06, A61P 7/04

(54) **USE OF MICROPARTICLES FOR VACCINES AND THE RELEASE OF BIOLOGICALLY ACTIVE MOLECULES**

(30) Priority: 14.11.2007 ES 200703014
(71) Applicant: Universidad del Pais Vasco, 48940 Leioa Vizcaya (ES)
(72) Inventor: HERNÁNDEZ MARTÍN, Rosa María, E-01006 Vitoria (ES); IGARTUA OLAECHEA, Manuela, E-01006 Vitoria (ES); PEDRAZ MUÑOZ, José Luis, E-01006 Vitoria (ES)
(74) Representative: ABG Patentes, S.L.
(86) International application number: PCT/ES2008/000717
(87) International publication number: WO 2009/063116

(57) **Abstract**

The invention relates to microparticles based on a PLGA biodegradable polymer and an alginate polymer which encapsulate immunologically active peptides or proteins. Said microparticles, and the pharmaceutical compositions developed from them and the uses thereof, are applicable to the field of human and animal health as a vaccine and system for releasing biologically active molecules.

## Description

### Field of the Invention

The invention relates to microparticles based on a PLGA-type biodegradable polymer and an alginate polymer encapsulating immunologically active peptides or proteins. Said microparticles, as well as the pharmaceutical compositions derived from them and their uses, have an application in the field of the human and animal health as a vaccine and as a release system for releasing biologically active molecules.

### Background of the Invention

The development of vaccines effective against infectious diseases represents a unique challenge for the scientific community. It is important to remember, however, that it is necessary to use new adjuvants and release systems improving the immunogenic capacity of antigens.

Vaccines (from Latin *vaccinus-a-um,* bovine; from *vacca-ae*, cow) are antigen preparations which once inside the organism cause an attack response, called an antibody (which eliminates the antigen). This response generates immunological memory, in most cases causing permanent immunity against the disease.

It is generally considered that there are four traditional types of vaccines:
- Inactivated vaccines: harmful microorganisms which have been treated with chemicals or heat and are no longer harmful.
- Attenuated live vaccines: microorganisms which have been cultured under specific conditions so that they loose their harmful properties.
- Toxoid vaccines: these are inactivated toxic components from microorganisms in cases in which these components are what cause the disease rather than the microorganism itself.
- Subunit vaccines: these are fragments of a microorganism which can generate an immune response.

Immunological adjuvants were originally described as substances which, used in combination with a certain antigen, cause a more intense immune response than when only the antigen is administered. The only adjuvant approved for use in humans are aluminum salts, generically referred to as Alum; this substance has a good safety profile but various studies conducted with different protein vaccines demonstrate that their adjuvant capacity is rather limited and it is also a proven fact that the induced response is fundamentally humoral, i.e. Th2 (Gupta et al. 1998, Adv. Drug Del, Rev. 32: 155-172). Furthermore Alum is not effective in inducing an immunological response at the mucosal level. Finally, it has been observed that IgE, which has been associated with allergic reactions, can also induce such response.

Various studies have clearly shown that PLGA microparticles represent an interesting strategy as a carrier for vaccine administration, since they control the rate of release of the encapsulated antigen, which allows simulating repeated injections of conventional schedules with a single immunization dose (Men et al. 1996, Vaccine 14: 1442-1450; Igartua et al. 1998, J. Control. Rel. 56: 63-73).

In addition, microparticles confer stability to the antigen, protecting it against enzymatic hydrolysis and gastric acid pH, and allowing the presence of the antigen intact in the site of deposition, as well as its administration through mucosal routes. Finally, the particle form of the antigen favors its uptake by antigen-presenting cells (APCs), such as for example dendritic cells, from which the immune response commences. The immunization studies conducted with PLGA microparticles have demonstrated that they are able to achieve suitable antibody response, cytotoxic T-cell (CTL) activity, and to induce mixed Th1/Th2 responses.

In addition, there are alginates. Alginates are natural polysaccharide copolymers formed by mannuronic and glucuronic acids. Although synthetic polymers are better than natural polymers in terms of purity and reproducibility, the ultrapurification of alginates confers low toxicity to them, being considered biocompatible. In addition to the important role of alginates in the textile and food industry, their similarity to the extracellular matrix (ECM) together with their biodegradability and biocompatibility allows them to be widely used as biomaterials in tissue engineering, cell therapy by means of the immobilization thereof and as controlled release systems for the controlled release of various therapeutic agents. Like PLGA, a number of works have described the use of alginates as polymer matrixes for the microencapsulation of peptides and proteins. Examples of said works are listed in European patent EP 1 079 811.

The latest trends in vaccine development indicate that the antigen uptake by dendritic cells is more efficient when the surface characteristics of the microparticulate systems are designed according to the so-called pathogen-associated molecular patterns which are small molecule sequences which pathogens usually have on their surface, such as for example bacterial lipopolysaccharide (LPS), peptidoglycans, nucleic acids, CpG and RGD sequences. RGD sequences are sequences of amino acids (arginine, glycine and aspartic acid) which act as receptors specific for integrins and are able to induce receptor-mediated phagocytosis.

There is currently a wide range of synthetic antigens of a peptide or protein nature which have the limitation of their low immunogenicity, their joint and repeated administration with adjuvant substances to obtain an effective immune response being necessary.

In recent decades, biodegradable polymer microparticles have become a promising release system for releasing peptide and protein antigens for obtaining vaccines against various infectious diseases:
- Document WO951101 describes methods and compositions for the encapsulation of antigens in PLGA microspheres for use as vaccines.
- Document ES2190350A1 describes a vaccine composition for brucellosis comprising poly(ε-caprolactone) microparticles as an adjuvant.
- Document WO00/50050 describes non-parenteral polymer multiparticulate formulations as a delivery system for delivering prophylactic and diagnostic therapeutic agents across mucosal membranes.
- The document by Jin, Xiao Bing; et al. Ectopic neocartilage formation from predifferentiated human adipose derived stem cells induced by adenoviral-mediated transfer of hTGF beta2. Biomaterials 2007, Vol. 28 (19), 2994-3003, presents alginate microspheres embedded in PLGA films for application in tissue engineering.
- The document by Liu, Xiaohua; et al. Novel polymeric microspheres containing norcantharidin for chemoembolization. J. Control. Rel. 2006, Vol. 116 (1), 35-41, describes PLGA-alginate microspheres for application in chemoembolization for tumor treatment.
- The document by Wang, Ye; et al. Biodegradable and complexed microspheres used for sustained delivery and activity protection of SOD. J. Biomed. Mat. Res., Part B: Appl. Biomat. 2006, Vol.79B (1), 74-78, shows microspheres formed by at least one biodegradable polymer (PLGA; alginate or chitosan) and a protein (superoxide dismutase).

### Summary of the Invention

In one aspect, the invention relates to a microparticle comprising (i) a first PLGA [poly(lactic-glycolic) acid]-type biodegradable polymer and (ii) a second alginate polymer linked to a ligand specific for a cell surface receptor, encapsulating an immunologically active peptide or protein.

The uses of the microparticle described in the present invention, as well as the pharmaceutical composition and the kit containing it and the method for preparing said microparticle are additional aspects of the present invention.

In another aspect, the invention relates to a microparticle as described in the present invention for its use as a vaccine.

In other aspects, the invention relates to the use of a microparticle in producing a drug for stimulating and/or inducing the immune response in an individual or as a release system for releasing immunologically active peptides or proteins.

In another aspect, the invention relates to a pharmaceutical composition comprising the microparticle of the present invention and a pharmaceutically acceptable carrier which can optionally comprise a therapeutic agent.

Likewise, in other aspects, the invention relates to the pharmaceutical composition of the invention for its use as a vaccine and its use in producing a drug for stimulating the immune response in an individual or as a release system for releasing immunologically active peptides or proteins.

In another aspect, the invention relates to a kit comprising the microparticle of the invention. In other aspects, the invention relates to the kit of the invention for its use as a vaccine, the use of the kit in producing a drug for stimulating and/or inducing the immune response in an individual and the use of the kit as a release system for releasing immunologically active peptides or proteins.

In another aspect, the invention relates to a process for preparing a microparticle according to the invention comprising the steps of
a. preparing a solution of PLGA in an organic solvent,
b. preparing an aqueous solution of alginate linked to a ligand specific for a cell surface receptor,
c. emulsifying the solutions with the immunologically active peptide or protein,
d. adding the emulsion obtained in step c) to a solution containing calcium chloride, and
e. isolating the microparticles

In addition, the present invention also contemplates the possibility of obtaining microparticles in which the alginate is not linked to a ligand specific for a cell surface receptor, but that the microparticle alone has all the properties and characteristics of the previously described microparticle.

Therefore, in another aspect, the invention relates to a process for preparing a microparticle comprising the steps of
a. preparing a solution of PLGA in an organic solvent,
b. preparing an aqueous solution of alginate,
c. emulsifying the solutions with the immunologically active peptide or protein,
d. adding the emulsion obtained in step c) to a solution containing calcium chloride, and
e. isolating the microparticles.

The microparticle obtainable by the previously described method, in which the alginate is not linked to a ligand specific for a surface receptor, is an additional aspect of the present invention, as well as all the applications and uses derived therefrom, such as its use as a vaccine, in producing a drug for stimulating and/or inducing the immune response in an individual, as a release system for releasing immunologically active peptides and proteins, and forming part of pharmaceutical compositions and kit, including the uses of said compositions and kits.

Finally, in another aspect the invention relates to the use of the alginate as an adjuvant of an immunologically active peptide or protein.

### Brief Description of the Drawings

Figure 1 are scanning electron microscopy photographs of the different formulations of microparticles produced.
Figure 2 is a graph showing the antigen release profile for each formulation of microparticles loaded with SPf66 or S-3.
Figure 3 is a bar chart showing the profile of IgG antibody titers specific against SPf66.
Figure 4 is a bar chart showing the profile of IgG antibodies against S-3.
Figure 5 is a bar chart representing the IgG2a/IgG1 ratio against SPf66 9 weeks after immunization.
Figure 6 is a bar chart representing the IgG2a/IgG1 ratio against S-3 9 weeks after immunization.
Figure 7 is a bar chart showing the IFN-γ secretion 48 hours after the *in vitro* stimulation of the cell culture of the lymphatic nodes of the animals immunized with SPf66.
Figure 8 is a bar chart showing the IFN-γ secretion 48 hours after the *in vitro* stimulation of the cell culture of the lymphatic nodes of the animals immunized with S-3.

### Detailed Description of the Invention

The authors of the present invention have surprisingly observed that biocompatible and biodegradable polymer microparticles, such as microparticles formed by PLGA [poly(lactic-glycolic) acid] plus alginate linked to a ligand specific for a cell surface receptor, are able to not only increase the immune response against the antigen encapsulated in the microparticle, but they also allow the administration of immunologically active peptides and proteins through less invasive routes, such as the nasal or intradermal route, than the usual methods of administration.

Without wishing to be bound by any theory, it is believed that this effect is due to the presence of alginate in the microparticle which, in combination with PLGA, allows a greater encapsulation of low molecular weight synthetic peptides and a lower initial release of the peptide, furthermore achieving an increase of the induced immune response against the encapsulated peptide or protein (antigen), in addition to favoring antigen administration through non-invasive routes as a result of the mucoadhesiveness of alginates.

Therefore, in a first aspect, the invention relates to a microparticle, hereinafter microparticle [I] of the invention, comprising (i) a first PLGA [poly(lactic-glycolic) acid]-type biodegradable polymer and (ii) a second alginate polymer linked to a ligand specific for a cell surface receptor, encapsulating an immunologically active peptide or protein.

In the present invention "microparticle" is understood as that particle comprising a diameter less than 1 mm, preferably between 1 and 500 micrometers, between 1 and 0.9, between 0.9 and 0.8, between 0.8 and 0.7, between 0.7 and 0.6, between 0.6 and 0.5, between 0.5 and 0.4, between 0.4 and 0.3, between 0.3 and 0.2, between 0.2 and 0.1 or less than 0.1 mm in diameter.

The mean microparticle size is mainly affected by different technological factors of the process for manufacturing said microparticles, such as the concentration of the biodegradable polymer, concentration of the surfactant present in the external phase of the double emulsion and rate of stirring.

The first polymer forming part of the microparticle [I] of the invention is a PLGA-type biodegradable polymer. In the present invention, "biodegradable polymer" is understood as polymers which dissolve or degrade in a period of time that is acceptable for the desired application, in this case *in vivo* therapy, once they are exposed to a physiological solution with pH comprised between 4 and 9, at a temperature comprised between 25°C and 40°C.

This type of polymers includes poly(lactic-glycolic) acid or PLGA, a polymer formed from lactic acid and glycolic acid with the following structural formula

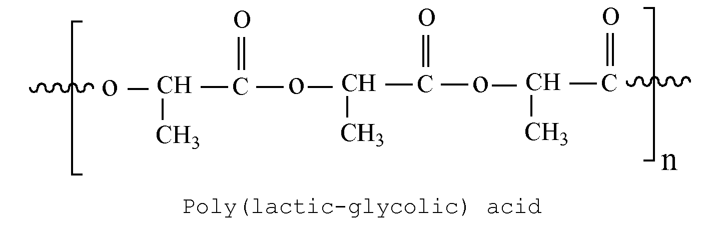

PLGA can have a different ratio of lactic acid and glycolic acid. Thus, PLGAs which can be used in the present invention preferably include 50:50 PLGA (50% lactic acid: 50% glycolic acid), 75:25 PLGA (75% lactic acid: 25% glycolic acid) or combinations of both.

The second polymer forming part of the microparticle [I] of the invention is an alginate polymer linked to a ligand specific for a cell surface receptor. Essentially, any alginate able to form a hydrogel is suitable for being used in the microparticles of the invention. Thus, the microparticles [I] of the invention can contain alginate mostly formed by mannuronic acid regions (MM blocks), by regions of guluronic acid (GG blocks) and by mixed sequence regions (MG blocks). The percentage and distribution of the uronic acids differ according to the origin of the alginate and contribute to the properties of the alginate, such as its viscosity. The person skilled in the art knows the percentages of each of the different blocks appearing in the different biological sources of alginates. Thus, the invention contemplates the use of alginates from *Laminaria hyperborea, Lessonia nigrescens, Lessonia trabeculata, Durvillaea antarctica, Laminaria digitata, Ecklonia maxima, Macrocystis pyrifera, Ascophyllum nodosum* and/or *Laminaria japonica* as well as mixtures of alginates of different species until achieving the desired content of GG, MM or GM blocks. By means of the use of a suitable combination of alginate polymers, a mixture can be obtained with a modulus of elasticity having a suitable value while the viscosity of the pre-gel solution is maintained at low enough levels to allow the suitable encapsulation/release of the immunologically active peptide or protein. Thus, the alginates which can be used in the present invention include GG alginates, MM alginates or combinations of both at a ratio of 90:10, 80:20, 70:30, 60:40, 50:50, 40:60, 30:70, 20:80 or 10:90. The results which are shown in the present invention have been obtained with an alginate of medium viscosity having more than 60% G blocks.

The invention also contemplates the use of alginates derived from the treatment of natural alginates with enzymes that are able to modify the integrating blocks to lead to alginates with improved properties. Thus, alginates resulting from the treatment of alginates with C5-epimerases, which convert M blocks into G blocks, as well as with the enzyme AlgE4 of the bacterium *Azotobacter vinelandii*, which is able to convert relatively rigid M blocks into MG blocks. The invention alternatively contemplates the use of alginates which have been modified by different physical treatments, particularly gamma rays, irradiation with ultrasounds or with ultraviolet light, as described by Wasikiewicz et al. (Rad. Phys. Chem. 2005, 73: 287-295).

Additionally, the alginate forming part of the microparticle [I] of the invention is linked to a ligand specific for a cell surface receptor. In the present invention, "ligand specific for a cell surface receptor" is understood as the molecule or peptide which is able to recognize a cell surface receptor and specifically bind to it. This ligand allows the microparticle [I] of the invention to be recognized by the receptors of the surface of specific cells, therefore it can bind thereto and interact with them. Essentially, any peptide having specific binding sites which can be recognized by cell surface receptors can be used in the present invention. Thus, the ligand specific for a cell surface receptor can be from cell adhesion molecules interacting with the extracellular matrix, such as fibronectin, the different members of the families of selectins, cadherins, lectins, integrins, immunoglobulins, collectins and galectins.

Thus, the ligand specific for a cell surface receptor used in the present invention can be a peptide derived from a region selected from the fibronectin regions involved in the binding with the integrins located in the cell membrane. For example, and without limiting the invention, said peptides derive from the tenth fibronectin type III repeat region containing the RGD peptide, from the fourteenth fibronectin type III repeat region containing the IDAPS peptide (SEQ ID NO: 3), from the fibronectin CSI region containing the LDV peptide and the fibronectin CS5 region containing the REDV peptide (SEQ ID NO: 4). These peptides can consist of fragments of the corresponding regions preserving their adhesive capacity, such as, for example, the QAGDV peptide (SEQ ID NO: 5) of fibrinogen, the LDV peptide of fibronectin and the IDSP peptide (SEQ ID NO: 6) of VCAM-I.

The present invention also contemplates the use of integrin-binding peptides as a ligand specific for a cell surface receptor, deriving from the tenth fibronectin type III repeat region comprising the RGD sequence, such as, for example, a peptide selected from the group of RGD, RGDS (SEQ ID NO: 7), GRGD (SEQ ID NO: 8), RGDV (SEQ ID NO: 9), RGDT (SEQ ID NO: 10), GRGDG (SEQ ID NO: 11), GRGDS (SEQ ID NO: 12), GRGDY (SEQ ID NO: 13), GRGDF (SEQ ID NO: 14), YRGDS (SEQ ID NO: 15), YRGDDG (SEQ ID NO: 16), GRGDSP (SEQ ID NO: 17), GRGDSG (SEQ ID NO: 18), GRGDSY (SEQ ID NO: 19), GRGDVY (SEQ ID NO: 20), GRGDSPK (SEQ ID NO: 21), CGRGDSPK (SEQ ID NO: 22), CGRGDSPK (SEQ ID NO: 23), CGRGDSY (SEQ ID NO: 24), cyclo-(RGDfK) (SEQ ID NO: 25), YAVTGRGD (SEQ ID NO: 26), AcCGGNGEPRGDYRAY-NH2 (SEQ ID NO: 27), AcGCGYGRGDSPG (SEQ ID NO: 28) and RGDPASSKP (SEQ ID NO: 29), cyclic variants of said peptides, multivalent variants both linear and branched (see for example Dettin et al. 2002, J. Biomed. Mater. Res. 60:466-471; Monaghan et al. 2001, Arkivoc, 2: U42-49; Thumshirn et al. 2003, Chemistry 9: 2717-2725; Scott et al, 2001, J. Gene Med. 3: 125-134) as well as combinations of two or more of said peptides.

Therefore, in another still more particular embodiment of the microparticle [I] of the invention, the ligand specific for a cell surface receptor is a peptide comprising the RGD sequence.

Likewise, the ligand specific for a cell surface receptor can be linked to the alginate polymer with different degrees of substitution, such that the concentrations of both components can vary and thus control the number of ligands specific for a cell surface receptor which are linked to the alginate polymer. Thus, the invention contemplates alginates containing between 1 and 100, between 100 and 200, between 200 and 300, between 300 and 400, between 400 and 500, between 500 and 600, between 600 and 700, between 700 and 800, between 800 and 900 and between 900 and 1000 molecules of said specific ligand for each polymer molecule.

As previously mentioned, the microparticle [I] of the invention can encapsulate an immunologically active peptide or protein. In the present invention, "immunologically active peptide or protein" is understood as that peptide or protein stimulating the immune response (humoral response, cell response or both) when it is introduced in an organism different from the organism from which said peptide or protein originates. Said immunologically active peptide or protein is generically referred to as antigen.

Therefore, in the present invention, the term antigen relates to any substance that can be recognized by the immune system of a subject and/or that can induce in a subject a humoral immune response or a cell immune response leading to the activation of B and/or T cells when it is introduced in a subject; by way of illustration, said term includes any native or recombinant immunogenic product obtained from a higher organism or from a microorganism, for example, a bacterium, a virus, a parasite, a protozoan, a fungus, etc., containing one or more antigenic determinants, for example, structural components of said organisms; toxins, for example, exotoxins, etc. Virtually any antigen can be used in producing microparticles of the invention loaded with antigen.

Examples of immunologically active peptides and proteins, without serving to limit the invention, include:
■ Peptides or proteins that are able (suitable or designed) to induce an immune response against an infectious disease, such as an infectious disease in animals caused by pathogenic microorganisms of animals, including humans, for example, infectious viruses, bacteria, fungi and parasites, relevant in human or animal health.

The proteins or peptides that are able to induce an immune response can be recombinant proteins or peptides, identical or similar to the native antigens of a specific microorganism.

Illustrative non-limiting examples of infectious viruses include viruses of the following families: Arteriviridae, Retroviridae, Picornaviridae, Caliciviridae, Togaviridae, Flaviviridae, Coronaviridae, Rhabdoviridae, Filoviridae, Paramyxoviridae, Orthomyxoviridae, Bunyaviridae, Arenaviridae, Reoviridae, Birnaviridae, Hepadnaviridae, Parvoviridae (parvovirus), Papovaviridae, Adenoviridae, Herpesviridae, Poxviridae, Iridoviridae, etc. Examples of antigens which can be used according to the present invention include, but are not limited to, HIV antigens, gp120 antigen, hepatitis B surface antigen, rotavirus antigens such as VP4 and VP7, influenza virus antigens such as hemagglutinin or nucleoprotein, the herpes simplex antigen thymidine kinase, etc.

Illustrative non-limiting examples of bacteria include both Gram-positive bacteria, e.g., *Pasteurella sp., Staphylococcus sp., Streptococcus sp.,* etc., and Gram-negative bacteria, e.g., *Escherichia coli, Pseudomonas sp., Salmonella sp*., etc. Specific examples of infectious bacteria include: *Helicobacter pylori, Borrelia burgdorferi, Legionella pneumophilia, Mycobacterium sp.* (e.g., *M. tuberculosis, M. avium, M. intracellulare, M. kansasii, M. gordonae*), *Staphylococcus aureus, Neisseria gonorrhoeae, Neisseria meningitidis, Listeria monocytogenes, Streptococcus pyogenes* (Group A Streptococcus), *Streptococcus agalactiae* (Group B Streptococcus), Streptococcus (viridans group), *Streptococcus faecalis, Streptococcus bovis, Streptococcus* (anaerobic species), *Streptococcus pneumoniae, Campylobacter sp., Enterococcus sp., Haemophilus influenzae, Bacillus anthracis, Corynebacterium diphteriae, Corynebacterium sp., Erysipelothrix rhusiopathiae, Clostridium perfringens, Clostridium tetani, Enterobacter aerogenes, Klebsiella pneumoniae, Pasteurella multocida, Bacteroides sp., Fusobacterium nucleatum, Streptobacillus moniliformis, Treponema pallidum, Treponema pertenue, Leptospira, Rickettsia, Actinomyces israelii, Chlamydia*, etc.

Illustrative non-limiting examples of infectious fungi include *Cryptococcus neoformans, Histoplasma capsulatum, Coccidioides immitis, Blastomyces dermatitidis, Chlamydia trachomatis* and *Candida albicans*.

The infectious parasites include by way of a non-limiting illustration protozoa, such as *Plasmodium sp.,* parasites that cause malaria, e.g. *P. falciparum, P. malariae, P. ovale, P. vivax,* etc., *Leishmania sp.,* parasites that cause leishmaniasis, e.g., *L. major, L. donovani, L. infantum, L. braziliensis, L. panamensis, L. mexicana,* etc., *Toxoplasma gondii, Schistosoma sp.,* etc., as well as parasitic nematodes, such as *Dirofilaria immitis,* etc. Examples of antigens for these parasites include the circumsporozoite antigen of *Plasmodium spp.;* the merozoite surface antigen of *Plasmodium spp.;* the SPf66 antigen of P. *falciparum;* S-3 antigen, molecule including four epitopes corresponding to different stages of the life cycle of P. *falciparum;* gp63 of *Leishmania spp.,* etc.
■ Peptides or proteins associated with tumors or cancers ("tumour markers") that are able (suitable or designed) to induce an immune response against a tumor or cancer cell, therefore the peptide or protein can be used in the treatment of cancers by means of the stimulation of an antigen-specific immune response against a tumor antigen.

Illustrative non-limiting examples of cancers which could be potentially treated according to the teachings of the present invention include bile duct cancer, brain cancer, breast cancer, cervical cancer, choriocarcinoma, colon cancer, endometrial cancer, esophageal cancer, stomach cancer, intraepithelial neoplasias, lymphomas, liver cancer, lung cancer (e.g., small cell and non-small cell lung cancer), melanoma, neuroblastomas, mouth cancer, ovarian cancer, pancreatic cancer, prostate cancer, rectal cancer, sarcomas, skin cancer, testicular cancer, thyroid cancer and renal cancer, as well as other carcinomas and sarcomas.

The selection of tumor antigens or antigenic determinants for the treatment of cancers can be done by a person skilled in the art in view of the state of the art (Renkvist et al. 2001, Cancer Immunol. Immunother. 50: 3-15), said antigens and antigenic determinants being included within the scope of the present invention. Representative examples of said antigens or antigenic determinants include: Her2 (breast cancer); GD2 (neuroblastoma); EGF-R (malignant glioblastoma); CEA (medullary thyroid cancer); CD52 (leukemia); human melanoma gp100 protein; human melanoma melan-A/MART-1 protein; tyrosinase; NA17-A nt protein; MAGE-3 protein; p53 protein; HPV16E7 protein; and antigenic fragments of said peptides or proteins.
■ Peptides or proteins that are able (suitable or designed) to induce an immune response against an allergen.

As it is used in this description, the term "allergen" relates to a peptide or protein to which a subject is sensitive and which causes an immune reaction, for example, allergenic extracts of pollens, allergenic extracts of insects, allergenic extracts of foods or food products, components present in saliva, pincers or stingers of insects inducing a sensitivity reaction in a subject, components present in plants inducing a sensitivity reaction in a subject, etc. Illustrative non-limiting examples of allergens include protein extracts of pollens, e.g., of *Lolium perenne, Poa pratense, Phleum pratense, Cynodon dactylon, Festuca pratensis, Dactylis glomerata, Secale cereale, Hordeum vulgare, Avena sativa, Triticum sativa, Artemisia vulgaris, Chenopodium album, Plantago lanceolata, Taraxacum vulgare, Parietaria judaica, Salsola kali, Urtica dioica, Olea europea, Platanus sp., Cupressus sp.,* etc.; protein extracts of insects, e.g., of *Dermatophagoides pteronyssinus, Dermatophagoides farinae, Acarus siro, Blomia tropicalis, Euroglyphus maynei, Glyciphagus domesticus, Lepidoglyphus destructor, Tyrophagus putrescentiae,* etc.; protein extracts of fungi or of animal epithelia, e.g., *Penicillium sp., Alternaria alternata, Cladosporium herbarum,* dog epithelium, cat epithelium, horse epithelium, etc.; protein extracts of foods or food products, etc.
■ Peptides or proteins that are able (suitable or designed) to induce an improved response against an autoantigen. As it is used herein, the term "autoantigen" relates to peptides or proteins encoded by the DNA of the subject and products generated by proteins or RNA encoded by the DNA of the subject. Examples of autoantigens are described in WO 02/56905.

As a result of the technical features of the microparticle [I] of the invention explained at the beginning of the present description, said microparticle is suitable for the release of immunologically active peptides or proteins in the organism and thereby stimulating the immune response of the organism against said peptides and proteins. Therefore, in another aspect, the invention relates to the microparticle [I] of the invention for its use as a vaccine.

In another aspect, the invention relates to the use of the microparticle [I] of the invention in producing a drug for stimulating and/or inducing the immune response in an individual.

In the present invention, "individual" or "subject" is understood as a member of an animal species, preferably a mammal and including but not limited to a domestic animal, a primate and a human; in the context of the present invention, the individual is preferably a male or female human of any race or age.

Due to the capacity of the microparticle of the invention to release the peptides and proteins encapsulated therein, the microparticle is very useful as a device for the release of peptide compounds with biological activity. Thus, in another aspect, the invention relates to the use of the microparticle [I] of the invention for the release of immunologically active peptides and proteins.

The release of the peptides and proteins can be done in a controlled manner by means of a suitable selection of the ratio of monomers (lactic acid and glycolic acid) forming the PLGA used in producing the microparticle. The degradation rate of these polymers fundamentally depends on the ratio of both monomers. Thus, by means of a suitable selection of said composition it is possible to achieve that the breakdown takes place during periods of time which can comprise from a few days up to several months. The molecular weight of the polymer also affects the degradation rate, because the greater the molecular weight the less time necessary for obtaining small hydrophilic monomers or oligomers (Lewis, et al. Controlled release of bioactive agents from lactide/glicolide polyesters. In: Chasin, M. et al. Biodegradable polymers as drug delivery systems. Ed. Marcel Decker, Inc. New York, 1990).

The microparticle [I] of the invention can be administered to the individual alone or forming part of pharmaceutical compositions together with a pharmaceutically acceptable carrier, and optionally along with the simultaneous or separate administration of a therapeutic agent. Therefore, in another aspect, the invention relates to a pharmaceutical composition, hereinafter, pharmaceutical composition [I] of the invention, comprising the microparticle [I] of the invention together with a pharmaceutically acceptable carrier. Pharmaceutically acceptable carriers used in producing pharmaceutical compositions can be found in the book "Rowe, R, Sheskey, P. and Owen, S. Handbook of Pharmaceutical Excipients. 5th Ed. APhA and Pharmaceutical Press. London, 2004".

After being administered to a subject, the microparticles [I] of the invention are able to release the immunologically active peptide or protein in a stable and gradual manner for prolonged times, such that they act like sustained-release drugs. Therefore, the microparticle [I] of the invention allows preventing the continuous and repeated administration of a therapeutic agent in those patients requiring baseline levels of said agent for a prolonged time.

The microparticle [I] of the invention or the pharmaceutical composition [I] of the invention can be administered to the patient parenterally, including intravascularly, intraperitoneally, subcutaneously, intradermally, etc. and non-parenterally, for example, topically, through the mucous membranes, i.e., orally, buccally, sublingually, nasally (by inhalation), vaginally, etc., and will be formulated in a dosage form suited for the chosen administration route, for example, in the form of sterile solutions, suspensions or lyophilized products in a suitable unit dosage form. Suitable excipients can be used, such as buffering agents, surfactants, preservatives, etc., can be used. Information about said excipients and pharmaceutical dosage forms of the microparticle [I] of the invention or the pharmaceutical composition [I] of the invention, can be found in the book "Rowe, R, Sheskey, P. and Owen, S. Handbook of Pharmaceutical Excipients. 5th Ed. APhA and Pharmaceutical Press. London, 2004", mentioned above.

The dose of microparticles [I] of the invention to be administered to an individual can vary within a broad range, for example, between approximately 0.01 and 100 mg per Kg of body weight.

Said pharmaceutical dosage forms can be manufactured by any of the chosen routes by means of conventional methods known by persons skilled in the art. By way of illustration, information about the processes for the manufacture of said pharmaceutical dosage forms of the protein of the invention can be found in the book "Swarbrick. J., Boylan, J.C. Encyclopedia of Pharmaceutical Technology. 2nd Ed. Marcel Dekker Inc. New York, 2002-2004".

In another particular embodiment, the pharmaceutical composition of the invention comprises the microparticle [I] of the invention, a pharmaceutically acceptable carrier and a therapeutic agent.

The term "therapeutic agent" includes any compound which can be used to treat, prevent or cure the diseases in a subject or that agent used to improve the physical and mental well-being in humans and animals. "Therapeutic agents" include but are not limited to chemotherapy agents, allergenic agents, analgesic agents, antitumor agents, a steroid, a retinoid, an antibiotic (antimicrobial, antiviral or antifungal) compound, an antioxidant, an anti-inflammatory compound, a neuroprotective agent, an anti-asthma agent, pulmonary surfactants, vitamin analogs, salicylic acid, etc. A therapeutic agent includes the pharmaceutically acceptable salts thereof, prodrugs and pharmaceutical derivatives thereof.

In another aspect, the invention relates to the pharmaceutical composition [I] of the invention for its use as a vaccine.

In another aspect, the invention relates to the use of the pharmaceutical composition [I] of the invention in producing a drug for stimulating and/or inducing the immune response of an individual.

In another aspect, the invention relates to the use of the pharmaceutical composition [I] of the invention as a release system for releasing immunologically active peptides or proteins.

As the person skilled in the art will understand, the microparticle [I] of the invention can be contained in a kit which can optionally comprise a therapeutic agent as has been previously defined.

Therefore, in another aspect, the invention relates to a kit, hereinafter kit [I] of the invention, comprising the microparticle [I] of the invention and furthermore comprising a therapeutic agent in a particular embodiment.

In the present invention, "kit" is understood as a product containing the microparticles [I] of the invention or the different components of the pharmaceutical composition [I] of the invention packaged so as to allow their transport, storage and their simultaneous or sequential administration. Thus, the kits of the invention can contain one or more suspensions, tablets, capsules, inhalers, syringes, patches and the like containing the microparticles [I] of the invention or the different components of the pharmaceutical composition [I] of the invention, and which can be prepared in a single dose or as multiple doses. The kit can additionally contain a carrier suitable for the resuspension of the microparticles [I] of the invention or the different components of the pharmaceutical composition [I] of the invention, such as aqueous media, e.g. saline solution, Ringer's solution, lactated Ringer's solution, dextrose and sodium chloride, water-soluble media, such as alcohol, polyethyleneglycol, propyl ethyleneglycol and water-insoluble carriers such as corn oil, cottonseed oil, peanut oil, sesame oil, ethyl oleate, isopropyl myristate and benzyl benzoate. Another component which can be present in the kit is a packing which allows maintaining the formulations of the invention within certain limits. Suitable materials for the preparation of such packing include glass, plastic include (polyethylene, polypropylene, polycarbonate and the like), flasks, vials, paper, sachets and the like.

The uses of the kit [I] of the invention are additional inventive aspects. Thus, in another aspect, the invention relates to the kit [I] of the invention for its use as a vaccine.

In another aspect, the invention relates to the kit [I] of the invention in producing a drug for stimulating and/or inducing the immune response in an individual.

In another aspect, the invention relates to the use of the kit [I] of the invention as a release system for releasing immunologically active peptides and proteins.

The microparticles can generally be obtained from biodegradable polymers which, in the case of the present invention, are PLGA and alginate polymers, by means of a process comprising dissolving said polymers in a suitable solvent (e.g., dichloromethane, ethyl acetate, acetone, water, etc.) and subsequently emulsifying them with an aqueous phase. This first emulsion is then added to a solution of a surfactant (e.g. polyvinyl alcohol) to form a double emulsion. Finally the desolvation or extraction of the solvent in which the PLGA was solubilized is performed after adding a suitable solvent, for example, a solvent or a mixture of solvents miscible with the solution of the biodegradable polymer, such as an isopropanolwater mixture, an aqueous suspension of microparticles being obtained from which the microparticles are isolated by conventional methods, for example, by means of vacuum filtration or centrifugation. The ratio by volume of organic phase:hydroalcoholic solution (isopropanol/water) may vary within a broad range, for example, between 1:10 and 1:100 (v:v).

The ratio of the immunologically active peptide or protein incorporated in the microparticle of the invention may vary within a broad range, for example, it can be of up to 25% by weight with respect to the total weight of the microparticles. Nevertheless, the suitable ratio will depend on the peptide or protein incorporated in each case.

The incorporation of the immunologically active peptide or protein to the microparticles of the invention can be done as described in WO 02/069938, by incorporating it into the solution of the biodegradable polymer (in the internal phase of the first emulsion) before the formation of microparticles, or subsequently adding it to the aqueous suspension of the already formed microparticles.

Therefore, in another aspect the invention relates to a process for preparing the microparticle [I] of the invention, comprising the steps of
a. preparing a solution of PLGA in an organic solvent,
b. preparing an aqueous solution of alginate linked to a ligand specific for a cell surface receptor,
c. emulsifying the solutions with the immunologically active peptide or protein,
d. adding the emulsion obtained in step c) to a solution containing calcium chloride, and
e. isolating the microparticles.

In a preferred embodiment of the process for preparing the microparticle [i] of the invention, the emulsion obtained in step c) can be added to a solution of a surfactant (e.g. polyvinyl alcohol) to obtain a double emulsion and next said double emulsion can be added to a hydroalcoholic solution containing calcium chloride to assure the complete gelling of the alginate.

As the person skilled in the art will understand, said process can comprise additional steps involving preferred embodiments of the process, such as for example, a step of evaporating/extracting the solvent to obtain the PLGA-alginate microparticles containing said immunologically active peptides and proteins, followed by a step of isolating the microparticles and optionally lyophilizing or drying the microparticles obtained.

The microparticles [I] of the invention can be purified or isolated by conventional methods, for example, by means of centrifugation, ultracentrifugation, tangential filtration, vacuum filtration, or evaporation, including the use of a vacuum.

In a particular embodiment of the process for preparing the microparticle [I] of the invention, the ligand specific for a cell surface receptor is a peptide comprising the RGD sequence.

Likewise, if desired, the microparticles [I] of the invention can be lyophilized by conventional methods. From a pharmacological point of view, it is important to have microparticles in lyophilized form given that this improves their stability during their long-term storage and preservation, in addition to reducing the volume of the product which will be handled. The microparticles [I] of the invention can be lyophilized in the presence of a common cryoprotectant such as glucose, sucrose, mannitol, trehalose, glycerol, lactose, sorbitol, polyvinylpyrrolidone, etc., preferably, sucrose or mannitol; at a concentration comprised within a broad range, preferably, between 0.1% and 20% by weight.

A cross-linking agent can optionally be added to improve the stability of the microparticles [I] of the invention, as described in WO 02/069938. Illustrative non-limiting examples of cross-linking agents which can be used include diaminated compounds, for example 1,3 diaminopropane, polysaccharides or simple saccharides, proteins and, generally, any molecule having functional groups that are able to react with the functional groups present in the biodegradable polymer.

Additionally, the invention also relates to a process for preparing a microparticle in which the alginate is not linked to a ligand specific for a cell surface receptor but which, due to the characteristics of the alginate in combination with the PLGA, preserves the properties of the microparticle [I] of the invention. Therefore, in another aspect, the invention relates to a process for preparing a microparticle, hereinafter microparticle [II] of the invention, comprising the steps of
a. preparing a solution of PLGA in an organic solvent,
b. preparing an aqueous solution of alginate,
c. emulsifying the solutions with the immunologically active peptide or protein,
d. adding the emulsion obtained in step c) to a solution containing calcium chloride, and
e. isolating the microparticles.

In a preferred embodiment of the process for preparing the microparticle [II] of the invention, the emulsion obtained in step c) can be added to a solution of a surfactant (e.g. polyvinyl alcohol) to obtain a double emulsion and next said double emulsion can be added to a hydroalcoholic solution containing calcium chloride to assure the complete gelling of the alginate.

As the person skilled in the art will understand, said process can comprise additional steps involving preferred embodiments of the process, such as for example, a step of evaporating/extracting the solvent to obtain the PLGA-alginate microparticles containing said immunologically active peptides and proteins, followed by a step of isolating the microparticles and optionally lyophilizing or drying the microparticles obtained.

As mentioned above, the microparticles [II] of the invention can be purified or isolated by conventional methods, for example, by means of centrifugation, ultracentrifugation, tangential filtration, vacuum filtration, or evaporation, including the use of a vacuum.

In a particular embodiment, the alginate linked or not to a specific ligand can be included in the external phase of the double emulsion and the rest of the manufacturing process would be the same as that described above.

Thus, in another aspect the invention relates to a microparticle obtainable by a process as previously described, in which the alginate of the resulting microparticle which is not linked to a ligand specific for a cell surface receptor, i.e., a microparticle, hereinafter microparticle [II] of the invention, comprising (i) a first PLGA [poly(lactic-glycolic) acid]-type biodegradable polymer and (ii) a second alginate polymer, encapsulating an immunologically active peptide or protein.

The different uses of the microparticle [II] of the invention are additional inventive aspects.

Therefore, in another aspect, the invention relates to the microparticle [II] of the invention for its use as a vaccine.

In another aspect, the invention relates to the use of the microparticle [II] of the invention in producing a drug for stimulating and/or inducing the immune response in an individual.

In another aspect, the invention relates to the use of the microparticle [II] of the invention as a release system for releasing immunologically active peptides or proteins.

In another aspect, the invention relates to a pharmaceutical composition comprising the microparticle [II] of the invention, hereinafter pharmaceutical composition [II] of the invention, and a pharmaceutically acceptable carrier.

In a particular embodiment, the pharmaceutical composition [II] of the invention furthermore comprises a therapeutic agent.

In another aspect, the invention relates to the pharmaceutical composition [II] of the invention for its use as a vaccine.

In another aspect, the invention relates to the use of the pharmaceutical composition [II] of the invention in producing a drug for stimulating and/or inducing the immune response of an individual.

In another aspect, the invention relates to the use of the pharmaceutical composition [II] of the invention as a release system for releasing immunologically active proteins or peptides.

In another aspect, the invention relates to a kit, hereinafter kit [II] of the invention, comprising the microparticle [II] of the invention.

In another aspect, the invention relates to the kit [II] of the invention for its use as a vaccine.

In another aspect, the invention relates to the use of the kit [II] of the invention in producing a drug for stimulating and/or inducing the immune response in an individual.

In another aspect, the invention relates to the use of the kit [II] of the invention in the release of immunologically active peptides or proteins.

In a final aspect, the invention relates to the use of the alginate as an adjuvant of an immunologically active peptide or protein.

The invention is illustrated below based on the following examples which are provided by way of a non-limiting illustration of the scope of the invention.

### EXAMPLE 1

### I. Production of the microparticles of the invention

50:50 PLGA, marketed by Boehringer Ingelheim G.K., Ingelheim, Germany, were used for the production of the microparticles. The alginate used was an MVG (medium viscosity, rich in G blocks) alginate of NovaMatrix, FMC Biopolymers Philadelphia, PA, USA, and the RGD alginate was obtained by means of modifying the MVG alginate following the process described by Rowley et al 1999. Biomaterials 20: 45-53.

The SPf66 and S3 peptides were obtained by means of solid phase synthesis following the methodology described by Merrifield (Merrifield, B. Solid phase peptide synthesis. I. Synthesis of Tetrapeptide. J. Am. Chem. Soc. 2149-2154. 1963) and modified by Houghten (Houghten R. General method for the rapid solid-phase synthesis of large number of peptides. Specificity of antigen-antibody interaction at the level of individual amino acids. Proc. Nat. Acad. Sci, 82: 5131-5135. 1985.), under Good Manufacturing Practice (GMP) conditions in the Fundación Instituto de Immunologia of Colombia. The amino acid sequence of the SPf66 peptide, in single-letter code, is the following: CGDELEAETQNVYAAPNANPYSLFQKEKMVLPNANPPANKKNAGC (SEQ ID NO: 1). The amino acid sequence of the S3 peptide is the following: DELEAETQNVYAAPNANPYSLFQKEKMVLPNANPPANKKNA (SEQ ID NO: 2).

The remaining reagents are analytical grade reagents and are obtained through common chemical distributors.

The microparticles (MP) were produced by means of the double emulsion solvent evaporation/extraction technique. Three formulations with different MPs were prepared for each antigen: PLGA MPs (PLGA MP), PLGA and 2% (w/w) alginate MPs (PLGA-alg MP), and PLGA and 2% alginate linked with RGD MPs (PLGA-alg RGD MP).
■ Preparation of the PLGA MPs
   An aqueous solution of the peptide at 10% (w/v) was added to another aqueous solution of dichloromethane with the PLGA polymer at 5% (w/v), emulsifying them by means of sonication for 30 seconds at 50 W (Branson® sonifier 250). This w/o emulsion was poured on an aqueous solution of polyvinyl alcohol at 8% (w/v), homogenizing them for 5 minutes a 8,000 rpm to favor the formation of a second w/o/w emulsion by means of a turbine homogenizer (Ultra-Turrax® T-25). Then the double emulsion was added to another aqueous solution of isopropanol at 2% (v/v) and was left to stir with a paddle stirrer (HEIDOLPH, Mod. RZR1) to favor the evaporation/extraction of the organic solvent and to thus induce the formation of the microparticles. After 1 hour, the MPs were collected by means of centrifugation at 10,000 rpm and were washed 3 times with distilled water by means of centrifugation and successive resuspension. Finally, the PLGA microparticles (PLGA MP) were resuspended in Milli-Q water and were lyophilized.
■ Preparation of the PLGA-alginate MPs
   The alginate MPs (PLGA-alg MP) were prepared in a manner similar to the PLGA MPs but with certain modifications. For the formation of the first w/o emulsion, the aqueous phase, in addition to the peptide at 10%, also contained the alginate at a ratio of 2% (w/w) with respect to the PLGA. This first emulsion was formed by means of sonication, and the second emulsion was formed by means of a turbine homogenizer. Once the w/o/w was obtained, it was added to 2% isopropanol and 0.6 mM CaCl₂ to assure the complete gelling of the alginate while at the same time evaporating the dichloromethane. The following steps are the same as for the previous formulation.
■ Preparation of the PLGA-alginate RGD MPs
   With respect to the PLGA-alginate linked to an RGD peptide microparticles (PLGA-alg RGD MP), they were produced exactly the same as PLGA-alg MP but using an alginate which has been bound to or linked with RGD (alg RGD).

### II. Microparticle Characterization

Once the microparticles were produced, they were completely characterized by means of determining their morphology, size, Z potential, percentage of encapsulated and surface-adsorbed peptide and *in vitro* release profile.

The morphological characterization was performed using the scanning electron microscopy (Jeol JSM-35 CF) technique. The particle size was determined by means of the laser ray diffraction technique using a COULTER^{®} COUNTER LS 130 particle analyzer. The modified Lowry assay, known as the bicinchoninic acid (micro-BCA) method (Smith et al. 1985, Anal. Biochem. 150: 76-85.) was used to determine the percentage of encapsulated peptide (w/w). The zeta potential was determined by means of laser Doppler velocimetry (Malvern^{®} Zetasizer 3000). The total encapsulated peptide was determined after the complete digestion of the microspheres with a solution of 0.2 M NaOH. The microparticle surface-adsorbed or associated peptide was determined by resuspending the microparticles in a 20 mM phosphate buffer solution, pH 7.4 and incubating at 37°C under continuous orbital stirring for 30 minutes. The microspheres subsequently settled by centrifugation and the SPf66 content in the supernatant was analyzed by means of the micro-BCA technique. The peptide associated with the surface corresponds to the percentage of the total encapsulated peptide which is present in the surface of the microparticles and therefore available for a quick release.

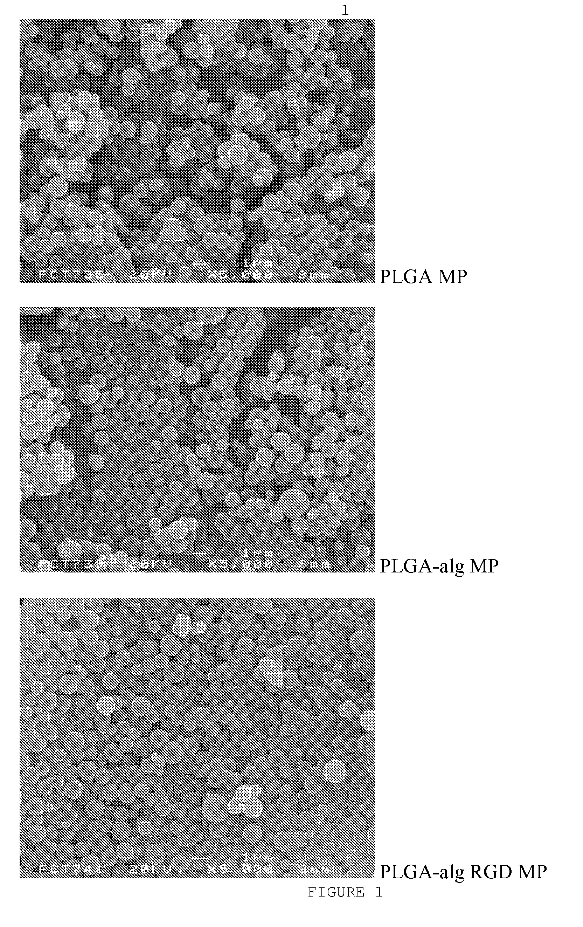

The analysis of the microparticles by means of scanning electron microscopy allowed observing their morphological characteristics, being smooth-surface spherical particles (Figure 1).

The different parameters characterized in the microparticles are included in Table 1. As can be observed in the table, the different types of MPs produced had a mean particle size of about 1 micrometer. However, the net surface charge of the microparticles is affected by the presence of alginate, since considerable differences were observed in the Zeta potentials of the MPs depending on the presence or not of alginate in the formulation. For both peptides (SPf66 and S-3), the MPs with alginate had more negative zeta potential values than their respective PLGA MPs. These results clearly show that the alginate (polyanion) is incorporated in the microparticles. With respect to the encapsulation efficiency (EE) and surface-adsorbed peptide (SAP), an increase in the EE is observed when the alginate is introduced, while the SAP was considerably reduced with respect to the microparticles prepared exclusively with PLGA (PLGA MP). The inclusion of alginate in the internal aqueous phase of the double emulsion, together with the peptide, allows increasing the viscosity of the first emulsion, making diffusion of the peptide towards the external aqueous phase difficult, which, together with the possibility of gelling the alginate by cross-linking with calcium chloride, prevents the loss of the peptide for the microparticle formation phase.

**Table 1**

| **Formulation** | **Encapsulation efficiency** | **Surface-adsorbed peptide (%)** | **Load (µg/mg MP)** | **Size (µm)** | **Zeta potential (mV)** |
|---|---|---|---|---|---|
| PLGA MP SPf66 | 78.91 | 17.96 | 71.74 | 1.02 | -8.0 |
| PLGA-alg MP SPf66 | 87.97 | 5.59 | 78.54 | 0.95 | -12.5 |
| PLGA-alg RGD MP SPf66 | 94.34 | 4.01 | 84.24 | 0.93 | -16.7 |
| PLGA MP S-3 | 82.72 | 37.73 | 75.20 | 0.82 | -9.8 |
| PLGA-alg MP S-3 | 87.07 | 6.04 | 77.74 | 0.89 | -11.3 |
| PLGA-alg RGD MP S-3 | 77.74 | 5.30 | 69.41 | 0.90 | -18.2 |

### In vitro Release Studies

For the purpose of obtaining the profile of the release of the peptide from the PLGA microparticles, exactly 20 mg of microspheres were weighed in test tubes and were resuspended in 20 mM phosphate buffer, pH 7.4, being incubated at 37°C in continuous orbital stirring. The samples were centrifuged at pre-established time intervals at 10,000 rpm for 10 minutes. The supernatant was extracted and analyzed to quantify the peptide by means of the micro-BCA technique described above. The removed supernatant is replaced with an equivalent volume of fresh buffer solution to continue with the release assay. The release profile of the peptide obtained for each microparticle formulation was expressed in terms of accumulated percentage of peptide released as a function of time. The release assays were conducted in triplicate for each of the produced formulations.

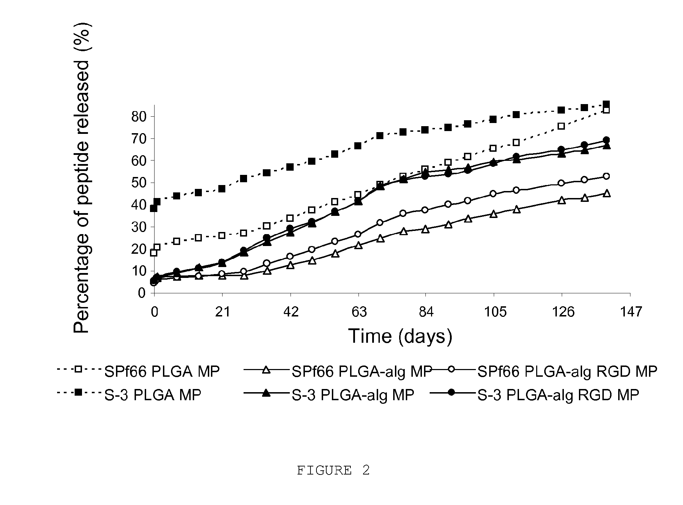

As can be observed in Figure 2, the profiles for the release of the peptides from the microparticles show a three-phase behavior. Differences in the release of the peptide are found only in the initial times (surface-associated peptide), where as the rest of the profile obtained for the 112 days the assay lasted shows very similar rates of release between the PLGA formulations and the 2 PLGA-alginate formulations for each of the peptides.

### III. Immunization Studies

The immune response induced by the microparticles was evaluated in Balb/c mice weighing 25 g and which were 6-8 weeks old, divided into 6 groups of 7 mice. Group 1 received an intradermal dose of 100 µg of the SPf66 antigen microencapsulated in 50:50 PLGA microspheres (PLGA MP). Group 2 received the same dose of SPf66 antigen microencapsulated in PLGA and 2% alginate microspheres (PLGA-alg MP). Group 3 received 100 µg of the SPf66 antigen microencapsulated in PLGA and 2% alginate linked with RGD microspheres (PLGA-alg RGD MP). Groups 4, 5 and 6 received the same dose of S3 antigen microencapsulated in the three types of microparticles described above, PLGA MP, PLGA-alg MP and PLGA-alg RGD MP, respectively.

### Determination of antibodies

Blood samples were taken from all the animals from the retro-orbital plexus under anaesthesia before the first immunization and at periodic intervals up to week 12. The blood samples were centrifuged at 5,000 rpm for 5 minutes, the serum was recovered and stored at -80°C for its subsequent immunochemical characterization. The levels of total antipeptide IgG antibodies, IgG2a and IgG1, were determined by means of a conventional ELISA technique.

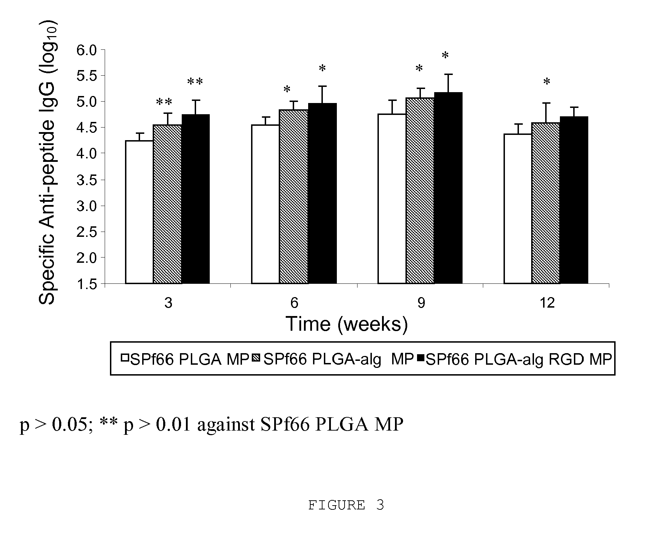

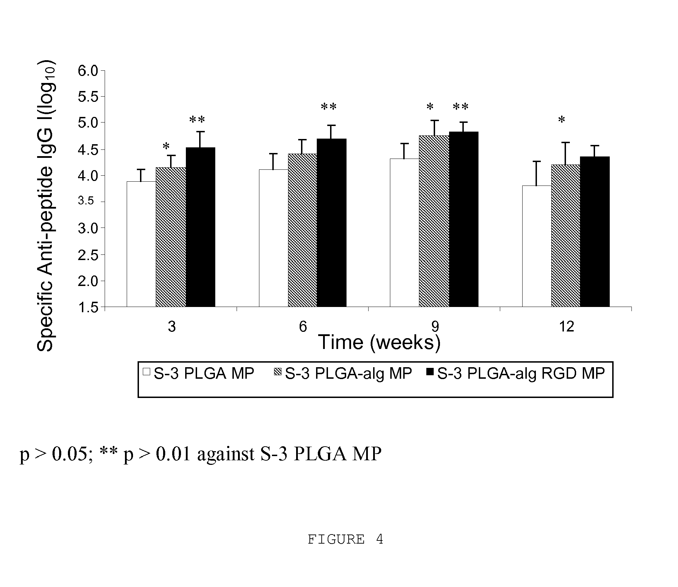

Figures 3 and 4 show the levels of antibodies (total IgG) obtained for the different groups of animals immunized with 100 µg of the SPf66 or S-3 antigen. As had been observed in previous studies, the PLGA microparticles (PLGA MP) loaded both with SPf66 and with S-3 generated a high humoral response when administered intradermally. It should also be pointed out that the microparticles with alginate (PLGA-alg MP) significantly improved the response for both peptide antigens. On the other hand, the incorporation of the RGD sequences in the alginate (PLGA-alg RGD MP) also showed differences between the particles formulated without alginate (PLGA MP), with the alginate not linked (PLGA-alg MP) or the algRGD (PLGA-alg RGD MP), especially in the initial times, 3 and 6 weeks, in which the PLGA-algRGD MPs induced IgG titers greater than those of the PLGA-alg MPs. The RGD sequences induce a specific recognition by the antigen-presenting cells (APCs), the latter being able to take up the MPs more efficiently.

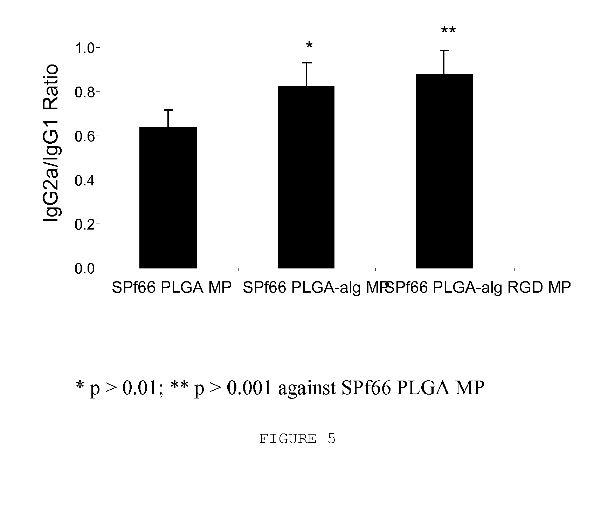

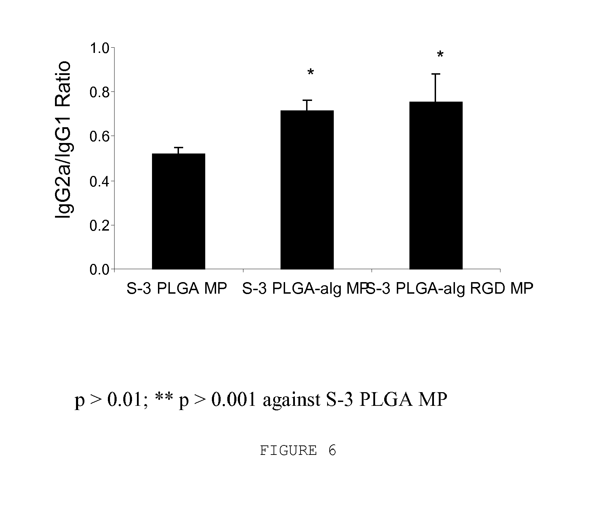

The production of antibody isotopes (IgG1 and IgG2a) by the immunized mice was studied at 9 weeks. All the formulations induced high and similar levels of IgG1. With respect to IgG2a, although all the groups showed detectable levels, differences were observed between the administered formulations. For both peptides, the MPs which incorporated alginate induced levels of IgG2a which were greater than those of the PLGA MPs. Furthermore, the MPs which contained the alginate modified with RGD sequences increased the secretion of this cytophilic isotype (IgG2a for mice) with respect to the MPs of the alginate not linked, said increase being greater for the case of the SPf66 peptide than for the S-3 peptide. This is very important because in infection caused by malaria, cytophilic antibodies play an important role in generating protection against the parasite. As can be observed in Figures 5 and 6, the IgG2a/IgG1 ratio was significantly greater for the MPs with alginate than for those produced exclusively with PLGA, for both peptides. The higher ratios correspond in both cases for the PLGA-alg RGD MP formulations.

### IFN-γ Secretion Assays

IFN-γ secretion from the cell culture of the lymphatic nodes extracted from the mice 14 weeks after immunization was determined by means of capture ELISA (OptEIA IFN-γ ELISA kit (Pharmingen, Becton Dickinson, San Diego, CA)).

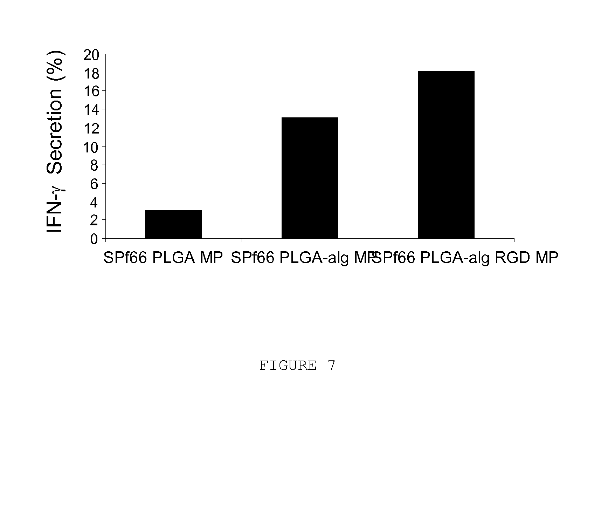

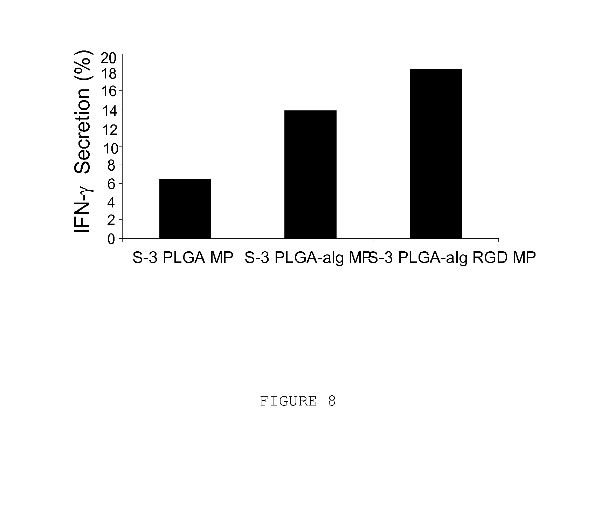

As shown in Figures 7 and 8, all the cell cultures of the lymphatic nodes of the groups immunized with the SPf66 or S-3 microparticles produced detectable levels of the IFN-γ cytokine after its *in vitro* stimulation with the peptide corresponding to the immunization. The groups which received the PLGA MPs induced the lowest levels of IFN-γ, whereas the highest levels were achieved by the PLGA-alg RGD MPs and the PLGA-alg MPs secreted levels that were intermediate between the levels of the other 2 formulations.

## Claims

1. A microparticle comprising (i) a first PLGA [poly(lactic-glycolic) acid]-type biodegradable polymer and (ii) a second alginate polymer linked to a ligand specific for a cell surface receptor, encapsulating an immunologically active peptide or protein.

2. The microparticle according to claim 1, wherein the ligand specific for a cell surface receptor is a peptide comprising the RGD sequence.

3. A microparticle according to claim 1 or 2 for its use as a vaccine.

4. The use of a microparticle according to claim 1 or 2 in producing a drug for stimulating and/or inducing the immune response in an individual.

5. The use of a microparticle according to claim 1 or 2 as a release system for releasing said immunologically active peptides or proteins.

6. A pharmaceutical composition comprising a microparticle according to claim 1 or 2 and a pharmaceutically acceptable carrier.

7. The composition according to claim 6 furthermore comprising a therapeutic agent.

8. A pharmaceutical composition according to claim 6 or 7 for its use as a vaccine.

9. The use of a pharmaceutical composition according to claim 6 or 7 in producing a drug for stimulating and/or inducing the immune response of an individual.

10. The use of a pharmaceutical composition according to claim 6 or 7 as a release system for releasing immunologically active peptides or proteins.

11. A kit comprising a microparticle according to claim 1 or 2.

12. The kit according to claim 11 furthermore comprising a therapeutic agent.

13. A kit according to claim 11 or 12 for its use as a vaccine.

14. The use of a kit according to claim 11 or 12 in producing a drug for stimulating and/or inducing the immune response in an individual.

15. The use of a kit according to claim 11 or 12 as a release system for releasing immunologically active peptides or proteins.

16. A process for preparing a microparticle according to claim 1 comprising the steps of
a. preparing a solution of PLGA in an organic solvent,
b. preparing an aqueous solution of alginate linked to a ligand specific for a cell surface receptor,
c. emulsifying the solutions with the immunologically active peptide or protein,
d. adding the emulsion obtained in step c) to a solution containing calcium chloride, and
e. isolating the microparticles.

17. The process according to claim 16, wherein the ligand specific for a cell surface receptor is a peptide comprising the RGD sequence.

18. A process for preparing a microparticle comprising the steps of
a. preparing a solution of PLGA in an organic solvent,
b. preparing an aqueous solution of alginate,
c. emulsifying the solutions with the immunologically active peptide or protein,
d. adding the emulsion obtained in step c) to a solution containing calcium chloride, and
e. isolating the microparticles.

19. A microparticle obtainable by a process according to claim 18.

20. A microparticle according to claim 19 for its use as a vaccine.

21. The use of a microparticle according to claim 19 in producing a drug for stimulating and/or inducing the immune response of an individual.

22. The use of a microparticle according to claim 19 as a release system for releasing said immunologically active peptides or proteins.

23. A composition comprising a microparticle according to claim 19 and a pharmaceutically acceptable carrier.

24. The composition according to claim 23 furthermore comprising a therapeutic agent.

25. A pharmaceutical composition according to claim 23 or 24 for its use as a vaccine.

26. The use of a pharmaceutical composition according to claim 23 or 24 in producing a drug for stimulating and/or inducing the immune response of an individual.

27. The use of a pharmaceutical composition according to claim 23 or 24 as a release system for releasing immunologically active peptides or proteins.

28. A kit comprising a microparticle according to claim 19.

29. A kit according to claim 28 for its use as a vaccine.

30. The use of a kit according to claim 28 in producing a drug for stimulating and/or inducing the immune response in an individual.

31. The use of a kit according to claim 18 in the release of immunologically active peptides or proteins.

32. The use of alginate as an adjuvant of an immunologically active peptide or protein.


SEQUENCE LISTING

<110>  Universidad de País Vasco (UPV/EHU)

<120>  USE OF MICROPARTICLES AS VACCINES AND THE RELEASE
       OF BIOLOGICALLY ACTIVE MOLECULES

<130>  P3646EPPC

<150>  ES P200703014.4
<151>  2007-11-14

<160>  29

<170>  PatentIn version 3.5

<210>  1
<211>  45
<212>  PRT
<213>  Plasmodium falciparum

<400>  1

Cys Gly Asp Glu Leu Glu Ala Glu Thr Gln Asn Val Tyr Ala Ala Pro
1               5                   10                  15

Asn Ala Asn Pro Tyr Ser Leu Phe Gln Lys Glu Lys Met Val Leu Pro
            20                  25                  30

Asn Ala Asn Pro Pro Ala Asn Lys Lys Asn Ala Gly Cys
        35                  40                  45


<210>  2
<211>  41
<212>  PRT
<213>  Artificial

<220>
<223>  Amino acid sequence of the S3 peptide

<400>  2

Asp Glu Leu Glu Ala Glu Thr Gln Asn Val Tyr Ala Ala Pro Asn Ala
1               5                   10                  15

Asn Pro Tyr Ser Leu Phe Gln Lys Glu Lys Met Val Leu Pro Asn Ala
            20                  25                  30

Asn Pro Pro Ala Asn Lys Lys Asn Ala
        35                  40


<210>  3
<211>  5
<212>  PRT
<213>  Artificial

<220>
<223>  Peptide derived from the fourteenth fibronectin type III repeat
       region

<400>  3

Ile Asp Ala Pro Ser
1               5


<210>  4
<211>  4
<212>  PRT
<213>  Artificial

<220>
<223>  Peptide derived from the fibronectin CS5 region

<400>  4

Arg Glu Asp Val
1


<210>  5
<211>  5
<212>  PRT
<213>  Artificial

<220>
<223>  Fragment of fibrinogen with adhesive capacity

<400>  5

Gln Ala Gly Asp Val
1               5


<210>  6
<211>  4
<212>  PRT
<213>  Artificial

<220>
<223>  Fragment of VCAM-I with adhesive capacity

<400>  6

Ile Asp Ser Pro
1


<210>  7
<211>  4
<212>  PRT
<213>  Artificial

<220>
<223>  Peptide derived from the tenth fibronectin type III repeat
       region

<400>  7

Arg Gly Asp Ser
1

<210>  8
<211>  4
<212>  PRT
<213>  Artificial

<220>
<223>  Peptide derived from the tenth fibronectin type III repeat
       region

<400>  8

Gly Arg Gly Asp
1


<210>  9
<211>  4
<212>  PRT
<213>  Artificial

<220>
<223>  Peptide derived from the tenth fibronectin type III repeat
       region

<400>  9

Arg Gly Asp Val
1


<210>  10
<211>  4
<212>  PRT
<213>  Artificial

<220>
<223>  Peptide derived from the tenth fibronectin type III repeat
       region

<400>  10

Arg Gly Asp Thr
1


<210>  11
<211>  5
<212>  PRT
<213>  Artificial

<220>
<223>  Peptide derived from the tenth fibronectin type III repeat
       region

<400>  11

Gly Arg Gly Asp Gly
1               5


<210>  12

<211>  5
<212>  PRT
<213>  Artificial

<220>
<223>  Peptide derived from the tenth fibronectin type III repeat
       region

<400>  12

Gly Arg Gly Asp Ser
1               5


<210>  13
<211>  5
<212>  PRT
<213>  Artificial

<220>
<223>  Peptide derived from the tenth fibronectin type III repeat
       region

<400>  13

Gly Arg Gly Asp Tyr
1               5


<210>  14
<211>  5
<212>  PRT
<213>  Artificial

<220>
<223>  Peptide derived from the tenth fibronectin type III repeat
       region

<400>  14

Gly Arg Gly Asp Phe
1               5


<210>  15
<211>  5
<212>  PRT
<213>  Artificial

<220>
<223>  Peptide derived from the tenth fibronectin type III repeat
       region

<400>  15

Tyr Arg Gly Asp Ser
1               5


<210>  16
<211>  6
<212>  PRT

<213>  Artificial

<220>
<223>  Peptide derived from the tenth fibronectin type III repeat
      region

<400>  16

Tyr Arg Gly Asp Asp Gly
1               5


<210>  17
<211>  6
<212>  PRT
<213>  Artificial

<220>
<223>  Peptide derived from the tenth fibronectin type III repeat
      region

<400>  17

Gly Arg Gly Asp Ser Pro
1               5


<210>  18
<211>  6
<212>  PRT
<213>  Artificial

<220>
<223>  Peptide derived from the tenth fibronectin type III repeat
      region

<400>  18

Gly Arg Gly Asp Ser Gly
1               5


<210>  19
<211>  6
<212>  PRT
<213>  Artificial

<220>
<223>  Peptide derived from the tenth fibronectin type III repeat
      region

<400>  19

Gly Arg Gly Asp Ser Tyr
1               5


<210>  20
<211>  6
<212>  PRT
<213>  Artificial

<220>
<223>  Peptide derived from the tenth fibronectin type III repeat
       region

<400>  20

Gly Arg Gly Asp Val Tyr
1               5


<210>  21
<211>  7
<212>  PRT
<213>  Artificial

<220>
<223>  Peptide derived from the tenth fibronectin type III repeat
       region

<400>  21

Gly Arg Gly Asp Ser Pro Lys
1               5


<210>  22
<211>  8
<212>  PRT
<213>  Artificial

<220>
<223>  Peptide derived from the tenth fibronectin type III repeat
       region

<400>  22

Cys Gly Arg Gly Asp Ser Pro Lys
1               5


<210>  23
<211>  8
<212>  PRT
<213>  Artificial

<220>
<223>  Peptide derived from the tenth fibronectin type III repeat
       region

<400>  23

Cys Gly Arg Gly Asp Ser Pro Lys
1               5


<210>  24
<211>  7
<212>  PRT
<213>  Artificial

<220>
<223>  Peptide derived from the tenth fibronectin type III repeat

region

<400>  24

Cys Gly Arg Gly Asp Ser Tyr
1               5


<210>  25
<211>  5
<212>  PRT
<213>  Artificial

<220>
<223>  Cyclic peptide derived from the fibronectin type III tenth repeat
       region

<400>  25

Arg Gly Asp Phe Lys
1               5


<210>  26
<211>  8
<212>  PRT
<213>  Artificial

<220>
<223>  Peptide derived from the tenth fibronectin type III repeat
       region

<400>  26

Tyr Ala Val Thr Gly Arg Gly Asp
1               5


<210>  27
<211>  14
<212>  PRT
<213>  Artificial

<220>
<223>  Peptide derived from the tenth fibronectin type III repeat
       region


<220>
<221>  MOD_RES
<222>  (1)..(1)
<223>  Acetylation

<220>
<221>  MOD_RES
<222>  (14)..(14)
<223>  Amidation

<400>  27

Cys Gly Gly Asn Gly Glu Pro Arg Gly Asp Tyr Arg Ala Tyr
1               5                   10

<210>  28
<211>  11
<212>  PRT
<213>  Artificial

<220>
<223>  Peptide derived from the tenth fibronectin type III repeat
       region


<220>
<221>  MOD_RES
<222>  (1)..(1)
<223>  Acetylation

<400>  28

Gly Cys Gly Tyr Gly Arg Gly Asp Ser Pro Gly
1               5                   10


<210>  29
<211>  9
<212>  PRT
<213>  Artificial

<220>
<223>  Peptide derived from the tenth fibronectin type III repeat
       region

<400>  29

Arg Gly Asp Pro Ala Ser Ser Lys Pro
1               5